# EUROPÄISCHE PATENTSCHRIFT

(11) **EP 1 758 512 B1**
(45) Veröffentlichungstag und Bekanntmachung des Hinweises auf die Patenterteilung: **25.03.2009**
(21) Anmeldenummer: 04737106.7
(22) Anmeldetag: 22.06.2004
(51) Int. Cl.: A61B 17/72

(54) **INTRAMEDULLÄRER MARKNAGEL**
INTRAMEDULLARY NAIL
CLOU INTRAMEDULLAIRE

(43) Veröffentlichungstag der Anmeldung: 07.03.2007
(73) Patentinhaber: Synthes GmbH, 4436 Oberdorf (CH)
(72) Erfinder: SCHLIENGER, André, CH-4144 Arlesheim (CH); BUETTLER, Markus, CH-4702 Oensingen (CH); SENN, Peter, CH-4437 Waldenburg (CH)
(74) Vertreter: Lusuardi, Werther
(86) Internationale Anmeldenummer: PCT/CH2004/000379
(87) Internationale Veröffentlichungsnummer: WO 2005/122931

(56) Entgegenhaltungen:
- EP-A- 0 882 431
- WO-A-03/101320
- WO-A-20/04082494
- US-A- 5 374 235
- US-B1- 6 197 065

## Beschreibung

Die Erfindung betrifft einen intramedullären Marknagel gemäss dem Oberbegriff des Patentanspruchs 1, insbesondere einen Marknagel für die distale Tibia.

Ein gattungsgemässer Marknagel ist aus der EP 1 024 762 LEU bekannt. Dieser bekannte Marknagel umfasst mehrere distale Querbohrungen, deren Bohrungsachsen alle die Zentrallinie des Marknagels schneiden. Nachteilig an dieser Anordnung der Querbohrungen ist, dass die Krafteinleitung für die durch den Marknagel zu übertragenden Kräfte in einem Knochenvolumen erfolgt, dessen Abmessungen quer zur Zentralachse auf den Durchmesser der Verriegelungsschrauben beschränkt ist und somit jeweils die gleichen Knochenfasern in Längsrichtung beansprucht werden.

Eine Femurkomponente für eine Hüftprothese, welche mehrere distale Querbohrungen umfasst, deren Bohrungsachsen die Zentrallinie der Femurkomponente nicht schneiden ist aus der US 6,197,065 MARTIN bekannt.

Hier will die Erfindung Abhilfe schaffen. Der Erfindung liegt die Aufgabe zugrunde, einen Marknagel zu schaffen, der im distalen Bereich eine hohe Stabilität der Verriegelung und eine optimal auf dem Knochenquerschnitt verteilte Krafteinleitung für die durch den Marknagel zu übertragenden Kräfte gestattet.

Die Erfindung löst die gestellte Aufgabe mit einem Marknagel, welcher die Merkmale des Anspruchs 1 aufweist.

Die durch die Erfindung erreichten Vorteile sind im wesentlichen darin zu sehen, dass dank des erfindungsgemässen Marknagels:
- die Stabilität der Versorgung durch die zusätzliche Asymmetrie der distalen Verriegelung erhöht wird;
- die Krafteinleitung der durch den Marknagel zu übertragenden Kräfte optimal auf dem Knochenquerschnitt verteilt ist; und
- nicht die gleichen Knochenfasern in Längsrichtung belastet werden.

Bei einer besonderen Ausführungsform weisen die Bohrungsachsen von mindestens zwei Querbohrungen Abstände d₁ > 0, respektive d₂ > 0 von der Zentrallinie auf.

Bei einer weiteren Ausführungsform laufen die Bohrungsachsen der mindestens zwei

Querbohrungen auf entgegengesetzten Seiten an der Zentrallinie vorbei. Der Vorteil dieser Ausführung liegt darin begründet, dass die in die beiden Querbohrungen einführbaren Knochenschrauben nicht die gleiche Knochenfaser des Röhrenknochens beanspruchen.

Bei einer weiteren Ausführungsform liegt die Bohrungsachse der mindestens einen Querbohrung mit dem Abstand d₁ in einer zur Zentrallinie orthogonalen Ebene.

Bei einer weiteren Ausführungsform liegen die Abstände d₁, respektive d₂ relativ zum Durchmesser D im Bereich von 0,0001 D < d < 0,6000 D, vorzugsweise im Bereich von 0,2D<d<0,5D.

Bei einer weiteren Ausführungsform weist der Marknagel eine zur Zentrallinie koaxiale Kannulierung auf.

Die orthogonalen Querschnittsflächen des Marknagels können vorzugsweise kreisförmig oder kreisringförmig ausgebildet sein.

Bei einer besonderen Ausführungsform sind die mindestens zwei Querbohrungen in der distalen Hälfte des Marknagels positioniert.

Der Abstand d₁ ist vorteilhafterweise grösser als 0,5 mm und vorzugsweise grösser als 1,0 mm. Der Abstand d₁ ist aber zweckmässigerweise kleiner als 5,0 mm und vorzugsweise kleiner als 3,5 mm. Der Abstand d₁ ist bis zu einem gewissen Grad vom Durchmesser des Marknagels abhängig. Das Verhältnis D/d₁ zwischen dem Durchmesser D des Marknagels und dem Abstand d₁ sollte deshalb zweckmässigerweise grösser als 5, vorzugsweise grösser als 8 sind. Anderseits sollte das Verhältnis D/d₁ zwischen dem Durchmesser D des Marknagels und dem Abstand d₁ zweckmässigerweise kleiner als 25, vorzugsweise kleiner als 21 sein.

Bei einer weiteren Ausführungsform sind die Mantelflächen der mindestens zwei Querbohrungen vollständig im Inneren des Marknagels gelegen, d.h. die Querbohrungen öffnen sich nur beim Eintritt in und beim Austritt aus dem Marknagel nach aussen und verlaufen im übrigen vollständig innerhalb des Marknagels.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht eines Marknagels;
Fig. 2 eine Seitenansicht des Marknagels nach Fig. 1;
Fig. 3 eine gegenüber Fig. 2 um 90° gedrehte Seitenansicht des Marknagels nach Fig. 1;
Fig. 4 einen orthogonalen Querschnitt durch einen gegenüber dem Marknagel nach den Fig. 1 - 3 modifizierten Marknagel; und
Fig. 5 eine perspektivische Ansicht der virtuellen Bohrungszylinder einer weiteren Ausführungsform des Marknagels.

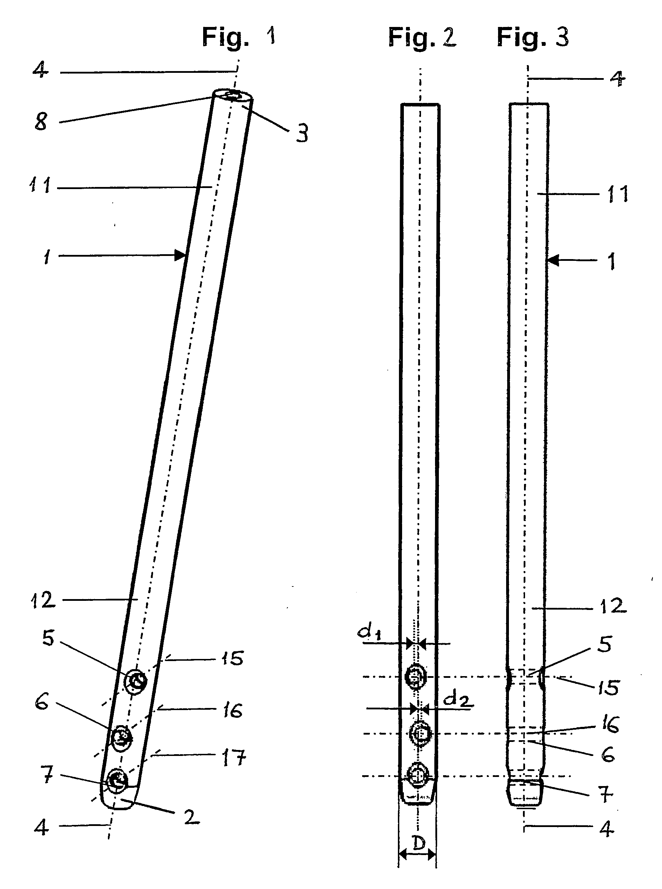

Das in den Fig. 1 - 3 dargestellte Beispiel eines Marknagels 1 besitzt eine proximale Hälfte 11, eine zur Einführung in den Markkanal geeignete, distale Hälfte 12 und eine Zentrallinie 4. Der Marknagel 1 weist einen im wesentlichen konstanten Durchmesser D auf und wird von einer Kannulierung 8 von seinem proximalen Ende 3 bis zu seinem distalen Ende 2 durchdrungen. Die distale Hälfte 12 weist drei, zur Aufnahme von Verriegelungsschrauben (nicht gezeichnet) geeignete Querbohrungen 5,6,7 auf. Die am proximalsten gelegene Querbohrung 5 besitzt eine Bohrungsachse 15, die mittlere Querbohrung 6 eine Bohrungsachse 16 und die distalste Querbohrung 7 eine Bohrungsachse 17. Die Querbohrungen 5;6;7 sind so angeordnet, dass ihre Bohrungsachsen 15;16;17 zueinander parallel sind. Der Durchmesser des Marknagels 1 beträgt D = 10 mm. Die Bohrungsachse 15 der Querbohrung 5 weist einen Abstand d₁ = 0,5 mm zur Zentrallinie 4 auf. Auch die Bohrungsachse 16 der Querbohrung 6 weist einen Abstand d₂ = 0,5 mm zur Zentralachse 4 auf, jedoch auf die entgegengesetzte Seite. Einzig die am distalsten angeordnete Querbohrung 7 weist eine die Zentrallinie 4 schneidende Bohrungsachse 17 auf. Die proximalste und die mittlere Querbohrung 5;6 sind nur soweit gegen den Umfang des Marknagels 1 verschoben, dass sie die äussere Mantelfläche des Marknagels 1 nicht durchbrechen.

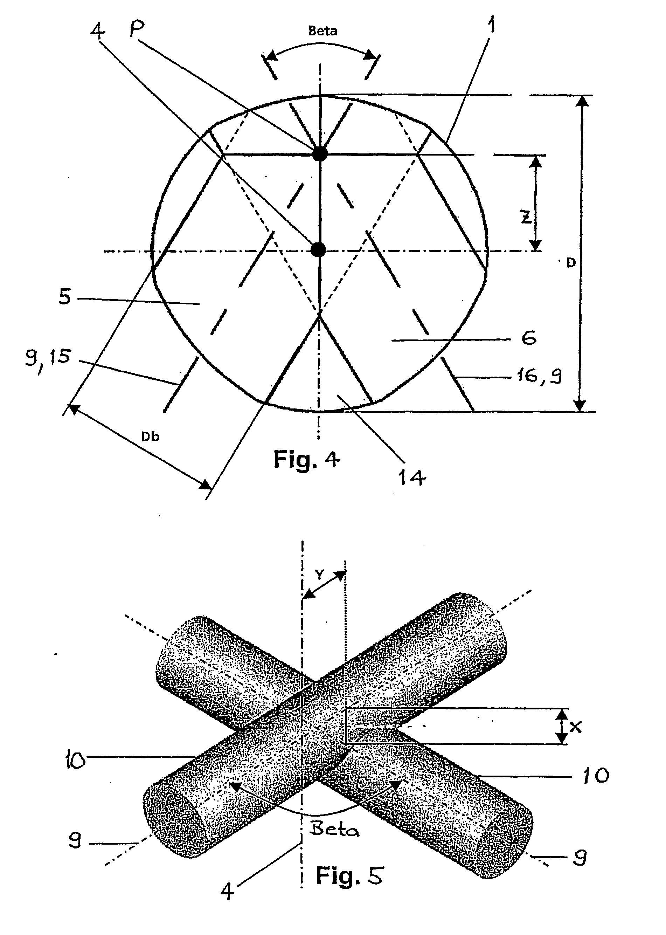

Fig. 4 zeigt ein Beispiel des Marknagels 1, welche sich von der in den Fig. 1 bis 3 dargestellten Ausführungsform darin unterscheidet, dass sich die virtuellen Bohrungszylinder 10 zweier benachbarter Querbohrungen 5;6 - gleich wie bei der Ausführungsform nach Fig. 5 - gegenseitig durchdringen. Die Zylinderachsen 9 der beiden virtuellen Bohrungszylinder 10 entsprechen den Bohrungsachsen 15; 16 der zwei Querbohrungen 5;6 und schneiden sich im Punkt P, welcher einen Abstand z = 0,4 D von der Zentrallinie 4 aufweist. Mit anderen Worten der Punkt P liegt nicht auf der Zentrallinie 4 des Marknagels 1. Die virtuellen Bohrungszylinder 10 weisen zwei getrennte Eingänge in den Marknagel 1 auf, aber nur einen gemeinsamen Ausgang aus dem Marknagel 1 heraus. Die Zylinderachsen 9 der beiden virtuellen Bohrungszylinder 10 liegen in einer zur Zentrallinie 4 orthogonalen Ebene, welche der Zeichenebene der Fig. 4 entspricht. Die Zylinderachsen 9 der beiden virtuellen Bohrungszylinder 10 können aber auch in einer Ebene liegen, welche von der Längsachse 4 unter einem von 90° abweichenden Winkel durchstossen wird.

Im gezeigten Beispiel schneiden sich die Zylinderachsen 9 der beiden virtuellen Bohrungszylinder 10 unter einem Winkel β von 60°. Der Durchmesser D_{b} der virtuellen Bohrungszylinder 10 beträgt im gezeigten Beispiel das 0,3-fache von D.

Fig. 5 zeigt eine Ausführungsform des Marknagels 1, bei welcher die Zylinderachsen 9 der beiden virtuellen Bohrungszylinder 10 auch windschief verlaufen können und einen kürzesten Abstand x aufweisen, welcher kleiner ist als die halbe Summe der beiden Durchmesser D_{b} der virtuellen Bohrungszylinder 10. Der kürzeste Abstand x zwischen den beiden windschiefen Zylinderachsen 9 verläuft im gezeigten Beispiel parallel zur Längsachse 4 und weist dazu einen kürzesten Abstand y > 0 auf. Die durch den kürzesten Abstand x definierte Strecke kann aber auch windschief zur Längsachse 4 verlaufen. Der Abstand y liegt im Bereich von D/2 > y > 0,4 D. Die Zylinderachsen 9 der beiden virtuellen Bohrungszylinder 10 scheiden sich hier unter einem Winkel β von 90°.

## Patentansprüche

1. Intramedullärer Marknagel (1) mit
A) einem distalen, zur Einführung In den Markraum geeigneten Ende (2), wobei der dem distalen Ende (2) zugewandte Abschnitt des Marknagels (1) einen Durchmesser D aufweist;
B) einem proximalen Ende (3);
C) mindestens zwei Querbohrungen (5;6) mit einer Bohrungsachse (15;16); und
D) einer Zentrallinie (4), gebildet durch die Verbindungslinie der Schwerpunkte der axial aufeinanderfolgenden, orthogonalen Querschnittsflächen (14) des Marknagels (1) ohne Berücksichtigung der Querbohrungen (5;6); wobei
E) die mindestens zwei Querbohrungen (5;6) in der distalen Hälfte des Marknagels (1) positioniert sind;
F) die Mantelflächen der mindestens zwei Querbohrungen (5;6) vollständig im inneren des Marknagels (1) gelegen sind; und
G) die Bohrungsachse (15) mindestens einer der Querbohrungen (5) einen Abstand d₁ > 0 von der Zentrallinie (4) aufweist;
**dadurch gekennzeichnet, dass**
H) die Bohrungsachsen (15;16) der mindestens zwei Querbohrungen (5;6) windschief zueinander verlaufen und einen kürzesten Abstand x aufweisen, welcher kleiner ist als die halbe Summe der beiden Durchmesser D_{b} der virtuellen Bohrungszylinder (10).

2. Marknagel (1) nach Anspruch 1, **dadurch gekennzeichnet, dass** die Bohrungsachsen (15;16) von mindestens zwei Querbohrungen (15;16) Abstände d₁> 0, respektive d₂ > 0 von der Zentrallinie (4) aufweisen.

3. Marknagel (1) nach Anspruch 2, **dadurch gekennzeichnet, dass** die Bohrungsachsen (15;16) der mindestens zwei Querbohrungen (5;6) auf entgegengesetzten Seiten an der Zentrallinie (4) vorbeilaufen.

4. Marknagel (1) nach einem der Ansprüche 1 bis 3, **dadurch gekennzeichnet, dass** die Bohrungsachse (15) der mindestens einen Querbohrung (5) mit dem Abstand d₁ in einer zur Zentrallinie (4) orthogonalen Ebene liegt.

5. Marknagel (1) nach einem der Ansprüche 1 bis 4, **dadurch gekennzeichnet, dass** die Abstände d₁, respektive d₂ relativ zum Durchmesser D im Bereich von 0,0001 D < d < 0,6000 D, vorzugsweise im Bereich von 0,2 D < d < 0,5 D liegen.

6. Marknagel (1) nach einem der Ansprüche 1 bis 5, **dadurch gekennzeichnet, dass** er eine zur Zentrallinie (4) koaxiale Kannulierung (8) aufweist.

7. Marknagel (1) nach einem der Ansprüche 1 bis 6, **dadurch gekennzeichnet, dass** die orthogonalen Querschnittsflächen (14) des Marknagels (1) kreisförmig oder kreisringförmig ausgebildet sind.

8. Marknagel (1) nach einem der Ansprüche 1 bis 7, **dadurch gekennzeichnet, dass** der Abstand d₁ grösser als 0,5 mm, vorzugsweise grösser als 1,0 mm ist.

9. Marknagel (1) nach einem der Ansprüche 1 bis 8, **dadurch gekennzeichnet, dass** der Abstand d₁ kleiner als 5,0 mm, vorzugsweise kleiner als 3,5 mm ist.

10. Marknagel (1) nach einem der Ansprüche 1 bis 9, **dadurch gekennzeichnet, dass** das Verhältnis D/d₁ zwischen dem Durchmesser D des Marknagels (1) und dem Abstand d₁ grösser als 5, vorzugsweise grösser als 8 ist.

11. Marknagel (1) nach einem der Ansprüche 1 bis 10, **dadurch gekennzeichnet, dass** das Verhältnis D/d₁ zwischen dem Durchmesser D des Marknagels (1) und dem Abstand d₁ kleiner als 25, vorzugsweise kleiner als 21 ist.

## Claims

1. Intramedullary nail (1) with
A) a distal end (2) suitable for its introduction into a medullary space, where the segment of the intramedullary nail (1) oriented toward the distal end (2) presents a diameter D;
B) a proximal end (3);
C) at least two cross holes (5; 6) with a borehole axis (15, 16); and
D) a central line (4) formed by a line connecting the centres of gravity of the axially successive, orthogonal cross-sectional areas (14) of the intramedullary nail (1) without taking into account the cross holes (5; 6);
E) the at least two cross holes (5; 6) are positioned in the distal half of the intramedullary nail (1);
F) the peripheral surfaces of the at least two cross holes (5; 6) are completely inside the intramedullary nail (1); and
G) the borehole axis (15) of at least one of the cross holes (5) presents a distance d1 > 0 from the central line (4),
**characterized in that**
H) the borehole axes (15, 16) of the at least two cross holes (5, 6) are skew relative to each other and present a shortest distance x, which is smaller than half of the sum of the two diameters D_{b} of the virtual borehole cylinders (10).

2. Intramedullary nail (1) according to claim 1, **characterized in that** the borehole axes (15; 16) of at least two cross holes (5; 6) present distances d₁ > 0 and d₂ > 0, from the central line (4), respectively.

3. Intramedullary nail (1) according to claim 2, **characterized in** fact that the borehole axes (15; 16) of the at least two cross holes (5; 6) pass the central line (4) on opposite sides.

4. Intramedullary nail (1) according to one of the claims from 1 to 3, **characterized in that** the borehole axis (15) of the at least one cross hole (5) lies in an orthogonal plane at a distance d₁ to the central line (4).

5. Intramedullary nail (1) according to one of the claims from 1 to 4, **characterized in that** the distances d₁ and d₂ to the diameter D are in a range of 0,0001 D < d < 0,6000 D, preferably in a range of 0,2 D < d < 0,5 D, respectively.

6. Intramedullary nail (1) according to one of the claims from 1 to 5, **characterized in that** it presents a channeling (8) coaxial to the central line (4).

7. Intramedullary nail (1) according to one of the claims from 1 to 6, **characterized in that** the orthogonal cross sectional surfaces (14) of the intramedullary nail (1) are conformed in a circular or circle-shaped form.

8. Intramedullary nail (1) according to one of the claims from 1 to 7, **characterized in that** the distance d₁ is larger than 0,5 mm, preferably larger than 1,0 mm.

9. Intramedullary nail (1) according to one of the claims from 1 to 8, **characterized in that** the distance d₁ is smaller than 5,0 mm, preferably smaller than 3,5 mm.

10. Intramedullary nail (1) according to one of the claims from 1 to 9, **characterized in that** the D/d₁ ratio between the diameter D of the intramedullary nail (1) and the distance d₁ is larger than 5, preferably larger than 8.

11. Intramedullary nail (1) according to one of the claims from 1 to 10, **characterized in that** the D/d₁ ratio between the diameter D of the intramedullary nail (1) and the distance d₁ is smaller than 25, preferably smaller than 21.

## Revendications

1. Clou intramédullaire (1) comportant
A) une extrémité distale (2) qui est appropriée pour être insérée dans l'espace médullaire, la portion du clou intramédullaire (1) qui se trouve du côté de l'extrémité distale (2) ayant un diamètre D ;
B) une extrémité proximale (3) ;
C) au moins deux perçages transversaux (5 ; 6) présentant un axe de perçage (15 ; 16) ; et
D) une ligne centrale (4) formée par la ligne de liaison des centres de gravité des sections orthogonales (14), axialement successives, du clou intramédullaire (1) sans tenir compte des perçages transversaux (5 ; 6) ;
E) les au moins deux perçages transversaux (5 ; 6) étant positionnés dans la moitié distale du clou intramédullaire (1) ;
F) les surfaces d'enveloppe des au moins deux perçages transversaux (5 ; 6) étant placées entièrement à l'intérieur du clou intramédullaire (1) ; et
G) l'axe de perçage (15) de l'au moins un des perçages transversaux (5) se trouvant à une distance d₁ > 0 de la ligne centrale (4) ;
**caractérisé en ce que**
H) les axes de perçage (15 ; 16) des au moins deux perçages transversaux (5 ; 6) s'étendant de façon gauche l'un par rapport à l'autre et étant écartés l'un de l'autre d'une très courte distance x qui est inférieure à la moitié de la somme des deux diamètres D_{b} des cylindres de perçage virtuels (10).

2. Clou intramédullaire (1) selon la revendication 1, **caractérisé en ce que** les axes de perçage (15 ; 16) d'au moins deux perçages transversaux (15 ; 16) sont écartés de la ligne centrale (4) d'une distance d₁ > 0 respectivement d₂ > 0.

3. Clou intramédullaire (1) selon la revendication 2, **caractérisé en ce que** les axes de perçage (15 ; 16) des au moins deux perçages transversaux (5 ; 6) s'étendent de part et d'autre de la ligne centrale (4).

4. Clou intramédullaire (1) selon l'une des revendications 1 à 3, **caractérisé en ce que** l'axe de perçage (15) de l'au moins un perçage (5) est situé à la distance d₁ dans un plan orthogonal à la ligne centrale (4).

5. Clou intramédullaire (1) selon l'une des revendications 1 à 4, **caractérisé en ce que** la distance d₁, respectivement d₂, est dans un rapport avec le diamètre D se trouvant dans le domaine 0,0001 D < d < 0,6000 D, de préférence dans le domaine 0,2 D < d < 0,5 D.

6. Clou intramédullaire (1) selon l'une des revendications 1 à 5, **caractérisé en ce qu'**il comporte une cannelure (8) qui est coaxiale à la ligne centrale (4).

7. Clou intramédullaire (1) selon l'une des revendications 1 à 6, **caractérisé en ce que** les sections orthogonales (14) du clou intramédullaire (1) ont une forme circulaire ou annulaire.

8. Clou intramédullaire (1) selon l'une des revendications 1 à 7, **caractérisé en ce que** la distance d₁ est supérieure à 0,5 mm, de préférence supérieure à 1,0 mm.

9. Clou intramédullaire (1) selon l'une des revendications 1 à 8, **caractérisé en ce que** la distance d₁ est inférieure à 5,0 mm, de préférence inférieure à 3,5 mm.

10. Clou intramédullaire (1) selon l'une des revendications 1 à 9, **caractérisé en ce que** le rapport D/d₁ entre le diamètre D du clou intramédullaire (1) et la distance d₁ est supérieure à 5, de préférence supérieure à 8.

11. Clou intramédullaire (1) selon l'une des revendications 1 à 10, **caractérisé en ce que** le rapport D/d₁ entre le diamètre D du clou intramédullaire (1) et la distance d₁ est inférieure à 25, de préférence inférieure à 21.
